# EUROPÄISCHE PATENTSCHRIFT

(11) **EP 3 210 564 B1**
(45) Veröffentlichungstag und Bekanntmachung des Hinweises auf die Patenterteilung: **29.07.2020**
(21) Anmeldenummer: 17163464.5
(22) Anmeldetag: 19.05.2011
(51) Int. Cl.: A61C 1/00, G01K 13/00, G01K 13/08, A61B 17/00

(54) **MEDIZINISCHES, INSBESONDERE DENTALES, HANDSTÜCK MIT TEMPERATURMESSVORRICHTUNG**
MEDICAL, IN PARTICULAR DENTAL, HANDPIECE WITH A TEMPERATURE MEASURING DEVICE
PIÈCE À MAIN MÉDICAL, EN PARTICULIER DENTAIRE, AVEC UN DISPOSITIF DE MESURE DE TEMPÉRATURE

(43) Veröffentlichungstag der Anmeldung: 30.08.2017
(62) Teilanmeldung aus: 11166638.4
(73) Patentinhaber: W & H Dentalwerk Bürmoos GmbH, 5111 Bürmoos (AT)
(72) Erfinder: PRUCKNER, Christian, 1190 Wien (AT)
(74) Vertreter: Benda, Ralf

(56) Entgegenhaltungen:
- EP-A1- 0 002 922
- WO-A1-2006/110670
- WO-A1-2009/072035
- WO-A2-2008/061225
- US-A1- 2009 259 244
- US-A1- 2009 322 541
- US-A1- 2010 055 642

## Beschreibung

Die vorliegende Erfindung betrifft ein medizinisches, insbesondere dentales, Handstück mit einer Temperaturmessvorrichtung zur Messung der Temperatur des Handstücks oder eines Teils des Handstücks.

Ein derartiges Handstück ist zum Beispiel aus der Patentschrift US 6,786,897 B2 bekannt. Das Handstück ist mit einer lösbaren Kappe oder mit einem Anstrich oder Lack versehen, die einen temperatur-sensitiven oder thermochromen Farbstoff enthalten, der eine Temperaturänderung durch einen Farbwechsel anzeigt.

Der Nachteil dieses Handstücks besteht unter anderem darin, dass der thermochrome Farbstoff die Temperaturänderung nur unpräzise und verzögert wiedergibt und dass die Temperaturmessung ausschließlich an jenen Bauteilen des Handstücks erfolgt, an denen der thermochrome Farbstoff angebracht ist.

Die Anmeldeschrift US 2009/0322541 A1 zeigt ein Handstück, dessen Kopfgehäuse mit einem skelett- oder rippenartigen Gürtel mit einem zentralen Band und davon abstehenden seitlichen Detektionsbändern versehen ist, an dem unter anderem Temperatursensoren vorgesehen sind. Die seitlichen Detektionsbänder übertragen die von unterschiedlichen Wärmequellen abgegebene Wärme mittels Wärmeleitung zu den Temperatursensoren.

Der Nachteil dieses Handstücks besteht unter anderem wiederum in der aufgrund der Wärmeleitung über die Detektionsbänder verzögerten und unpräzisen Temperaturmessung sowie in der durch die Anordnung der Detektionsbänder auf bestimmte Regionen oder Bauteile des Handstücks beschränkten Temperaturmessung.

Die Anmeldeschrift WO 2009/072035 A1 offenbart einen Herzkatheter mit einer korbartigen Vorrichtung aus Drähten und optischen Fasern zur Abgabe von Energie auf Herzgewebe. An dieser korbartigen Vorrichtung sind Temperatur-Messstellen vorgesehen, um die Temperatur des Herzgewebes bestimmen zu können. Die Temperatur-Messstellen umfassen Thermoelemente, die durch Kontakte der Drähte und Fasern gebildet sind. Über diese Drähte und Fasern wird einer Steuervorrichtung ein elektrisches Thermospannungssignal zur Verfügung gestellt, auf dessen Basis die Temperatur des Herzgewebes ermittelt werden soll.

Der vorliegenden Erfindung liegt daher die Aufgabe zugrunde ein medizinisches, insbesondere dentales, Handstück mit einer alternativen Temperaturmessvorrichtung zur Messung der Temperatur des Handstücks oder eines Teils des Handstücks zu schaffen, welches insbesondere einen oder mehrere der oben genannten Nachteile nicht aufweist.

Diese Aufgabe wird durch ein medizinisches, insbesondere dentales, Handstück nach Anspruch 1 gelöst, welches umfasst: eine Außenhülse, eine Antriebsvorrichtung zum in Bewegung Versetzen eines mit dem Handstück verbindbaren Werkzeugs und eine Temperaturmessvorrichtung zur Messung der Temperatur des Handstücks oder eines Teils des Handstücks.

Gemäß einem Ausführungsbeispiel sind mehrere Wärmequellen am oder im Handstück vorhanden, wobei insbesondere eine Wärmequelle das Handstück oder Teile des Handstücks stärker erwärmt als eine andere Wärmequelle oder wobei insbesondere eine Wärmequelle mehr Wärme oder Wärmestrahlung emittiert als eine andere Wärmequelle. Beispielsweise sind im Handstück oder im Handstückkopf zwei Lager, insbesondere Wälzlager, vorgesehen, wobei ein erstes Lager aufgrund eines Gebrechens oder aufgrund einer Verschmutzung wärmer wird als ein zweites Lager.

Selbstverständlich können die im Vorstehenden genannten Wärmequellen auch andere Bauteile des Handstücks als die genannten Lager umfassen, insbesondere jegliche relativ zueinander bewegte Bauteile des Handstücks, vorzugsweise bewegbare und stillstehende, einander kontaktierende Bauteile, zum Beispiel einen Werkzeughalter und eine Werkzeuglösevorrichtung des Handstücks oder Teile des Werkzeughalters und der Werkzeuglösevorrichtung oder einen Rotor eines Motors oder eines Generators oder die Außenhülse des Handstücks und ein relativ dazu bewegliches Bauteil des Handstücks. Eine Wärmequelle kann auch durch ein mit elektrischer Energie versorgbares Bauteil, wie zum Beispiel eine Beleuchtungsvorrichtung, einen Elektromotor, insbesondere dessen Spulen, eine elektrische oder elektronische Schaltung, einen Sensor oder Detektor etc. gebildet sein. Diese Aufzählung der möglichen Wärmequellen ist beispielhaft und nicht vollständig und trifft in entsprechender Weise auf alle weiteren beschriebenen Ausführungsbeispiele zu.

Der Innenraum des Handstücks ist vorzugsweise durch ein Fluid, insbesondere Luft, enthaltendes Volumen gebildet, welches Bauteile des Handstücks voneinander trennt, wobei in dem Volumen die Temperaturmessvorrichtung vorgesehen ist.

Erfindungsgemäß umfasst das medizinische, insbesondere dentale, Handstück eine Temperaturmessvorrichtung zur Messung der Temperatur des Handstücks oder eines Teils des Handstücks, wobei die Temperaturmessvorrichtung als elektrische Temperaturmessvorrichtung ausgebildet ist.

Ein Vorteil einer elektrischen Temperaturmessvorrichtung besteht unter anderem in der präzisen und raschen Ermittlung des Temperarturwerts.

Erfindungsgemäß weist die elektrische Temperaturmessvorrichtung zumindest ein Thermoelement auf, welches ausgebildet ist, zur Messung der Temperatur ein elektrisches Thermospannungssignal zur Verfügung zu stellen. Der Vorteil eines Thermoelements liegt insbesondere in seinem einfachen Aufbau und seiner Robustheit, insbesondere gegenüber externen Einflüssen, die zum Beispiel während der Reinigung oder Sterilisation des Handstücks auftreten. Vorzugsweise ist eine dem Thermoelement zugeordnete Auswerteeinheit vorgesehen, die ausgebildet ist, aus dem elektrischen Thermospannungssignal des Thermoelements einen Temperaturwert zu ermitteln.

Der Begriff Thermoelement umfasst eine Vorrichtung, die durch Thermoelektrizität Wärme in elektrische Energie wandelt. Insbesondere weist ein Thermoelement zwei unterschiedliche und an einem Ende miteinander verbundene, elektrisch leitende Materialien, insbesondere Metalle oder Metalldrähte auf, um damit Temperaturmessungen durchführen zu können. Ein Thermoelement weist somit bevorzugt einen bimetallischen Thermokontakt auf. Eine Temperaturdifferenz an den Enden der Metalle oder Metalldrähte erzeugt aufgrund des Seebeck-Effekts innerhalb der beiden Metalle eine elektrische Ladungstrennung oder eine elektrische Thermospannung. Diese elektrische Potentialdifferenz ist annähernd proportional zur Temperaturdifferenz an den Enden der Metalle. Um die absolute Temperatur an den Metallen oder Drahtenden bestimmen zu können, müssen weitere Maßnahmen ergriffen werden, zum Beispiel die Messung der Umgebungstemperatur, zu der die Temperaturdifferenz an den Enden der Metalle addiert wird. Da der Aufbau und die Funktion eines Thermoelements bekannt sind, wird darauf nicht weiter eingegangen.

Das zumindest eine Thermoelement umfasst vorzugsweise zwei elektrisch leitende Materialien, insbesondere ein Metall oder einen Metalldraht, zum Beispiel Stahl, Kupfer, eine Kupfer-Nickel-Legierung (Konstantan) oder andere Legierungen, welche zum Beispiel Nickel, Chrom, Eisen oder Kupfer enthalten.

Vorzugsweise ist das zumindest eine Thermoelement in einem Innenraum des Handstücks angeordnet, wobei der Innenraum durch ein Fluid, insbesondere Luft, enthaltendes Volumen gebildet ist, welches Bauteile des Handstücks voneinander trennt. In vorteilhafter Weise misst das zumindest eine Thermoelement damit insbesondere die Temperatur des Innentraums und / oder die (gemittelte) Temperatur mehrerer Wärmequellen, welche Wärme in den Innenraum abgeben, und / oder einen gemittelten Temperaturwert oder Durchschnittstemperaturwert, insbesondere einen gemittelten Temperaturwert oder Durchschnittstemperaturwert mehrerer Bauteile und / oder Wärmequellen des Handstücks und / oder eines die Temperaturmessvorrichtung umgebenden Raumvolumens, zum Beispiel eines Teilvolumens des Handstücks.

Erfindungsgemäß ist das zumindest eine Thermoelement an einem Bauteil des Handstücks vorgesehen, insbesondere an einer Wärmequelle oder an einem Wärme abgebenden Bauteil, wobei das Bauteil des Handstücks, an dem das Thermoelement vorgesehen ist, ein Material aufweist, welches Teil des Thermoelements oder der Thermoelementverbindung ist. Durch das Vorsehen des Thermoelements an einem Bauteil des Handstücks kann in vorteilhafter Weise insbesondere die Temperatur dieses Bauteils bestimmt werden. Durch das Verwenden eines Materials des Bauteils, an dem das Thermoelement angeordnet ist, als Teil des Thermoelements oder der Thermoelementverbindung kann in vorteilhafter Weise die Baugröße des Thermoelements verringert werden und kann, insbesondere wenn es sich um ein größeres Bauteil handelt, zum Beispiel um die Außenhülse des Handstücks, die Kontaktierung des Thermoelements, die das Thermospannungssignal ableitet, entfernt von der eigentlichen Thermoelementverbindung (d.h. dem Kontaktpunkt oder -bereich der beiden Materialien des Thermoelements) angeordnet sein, wodurch die Anordnung des Thermoelements bzw. der Kontakte und Leitungen zur Ableitung der Thermospannungssignal im beengten Inneren des Handstücks vereinfacht wird. Das Bauteil des Handstücks, an dem das Thermoelement vorgesehen ist und das ein Material aufweist, welches Teil des Thermoelements oder der Thermoelementverbindung ist, durch eine Außenhülse des Handstücks oder durch ein mit der Außenhülse des Handstücks (elektrisch leitend) verbundenes Bauteil des Handstücks, zum Beispiel einem Lager zur Lagerung eines Werkzeughalters für das mit dem Handstück verbindbare Werkzeug oder einem Kontaktring, gebildet.

Gemäß einem Ausführungsbeispiel, welches nicht Teil der Erfindung ist, umfasst die elektrische Temperaturmessvorrichtung ein Material, dessen elektrischer Widerstand oder dynamischer Widerstand in Abhängigkeit der Temperatur variabel ist. Das Material umfasst zum Beispiel einen Ohmschen Widerstand, einen elektrischen Leiter, insbesondere ein Metall, eine Keramik (ein gesintertes Metalloxid) oder einen dotierten Halbleiter. Bevorzugt ist zumindest eines dieser Materialien Teil eines Sensors der elektrischen Temperaturmessvorrichtung. Je nach Ausführung ist ein derartiger Sensor als aktiver Sensor, der zum Bestimmen der Temperatur eine Strom- oder Spannungsversorgung benötigt, oder als passiver Sensor ausgebildet, der zum Bestimmen der Temperatur keine Strom- oder Spannungsversorgung benötigt.

Der Begriff Handstück umfasst insbesondere mit einer Hand greifbare medizinische oder dentale Geräte, zum Beispiel gerade, pistolenförmige, gewinkelte oder gebogene Handgriffe, die im Dentalbereich oftmals als Winkelstücke bezeichnet werden, Teile von Handstücken oder Handgriffen, insbesondere einen Kopfabschnitt, der zum Beispiel mit einem davon lösbaren Griffabschnitten verbindbar ist, sowie Kupplungen, Adapter, Verbindungsstücke und Antriebseinheiten, zum Beispiel elektrische oder pneumatische Motoren. Unter der Bezeichnung Handstück werden des Weiteren sowohl schnurlose Handstück verstanden, insbesondere mit einer austauschbaren oder aufladbaren Energiequelle, als auch Handstück, die eine Versorgungsleitung und eine damit verbundene Steuer-, Regel- und / oder Versorgungseinheit umfassen. Gemäß einem bevorzugten Ausführungsbeispiel, welches auf alle genannten Ausführungsformen und -beispiele anwendbar ist, weist das Handstück einen Handstückkopf auf, in welchem ein Werkzeughalter für das mit dem Handstück verbindbare Werkzeug und die Temperaturmessvorrichtung, insbesondere der Temperatursensor der Temperaturmessvorrichtung, vorgesehen sind.

Die Erfindung wird nachfolgend anhand bevorzugter Ausführungsbeispiele und Bezug nehmend auf die beigefügten Zeichnungen erläutert:
Figur 1 zeigt eine Außenansicht eines Ausführungsbeispiels eines erfindungsgemäßen Handstücks mit einer Temperaturmessvorrichtung zur Messung der Temperatur des Handstücks oder eines Teils des Handstücks.
Figur 2 zeigt ein Ausführungsbeispiel eines Handstückkopfs mit einer Temperaturmessvorrichtung zur Messung der Temperatur des Handstücks oder eines Teils des Handstücks, wobei die Temperaturmessvorrichtung, die nicht Teil der Erfindung ist, ein Pyrometer oder einen Infrarotsensor umfasst.
Die Figuren 3 und 4 zeigen zwei Ausführungsbeispiele, die nicht Teil der Erfindung sind, von Handstückköpfen mit je einer Temperaturmessvorrichtung zur Messung der Temperatur des Handstücks oder eines Teils des Handstücks, wobei die Temperaturmessvorrichtungen jeweils einen optischen Leiter für elektromagnetische Strahlung umfasst, dessen Streuung oder Brechungsindex aufgrund von Temperaturänderungen variiert.
Die Figuren 5 - 9 zeigen Ausführungsbeispiele von Handstückköpfen mit je einer Temperaturmessvorrichtung zur Messung der Temperatur des Handstücks oder eines Teils des Handstücks, wobei die Temperaturmessvorrichtungen jeweils ein Thermoelement umfassen.
Figur 10 zeigt ein Ausführungsbeispiel eines Handstückkopfs mit einer Temperaturmessvorrichtung zur Messung der Temperatur des Handstücks oder eines Teils des Handstücks, wobei die Temperaturmessvorrichtung zwei Thermoelemente umfasst.
Die Figuren 11 - 13 zeigen Ausführungsbeispiele, die nicht Teil der Erfindung sind, von Handstückköpfen mit je einer Temperaturmessvorrichtung zur Messung der Temperatur des Handstücks oder eines Teils des Handstücks, wobei die elektrische Temperaturmessvorrichtung ein Material umfasst, dessen elektrischer Widerstand oder dynamischer Widerstand in Abhängigkeit der Temperatur variabel ist.

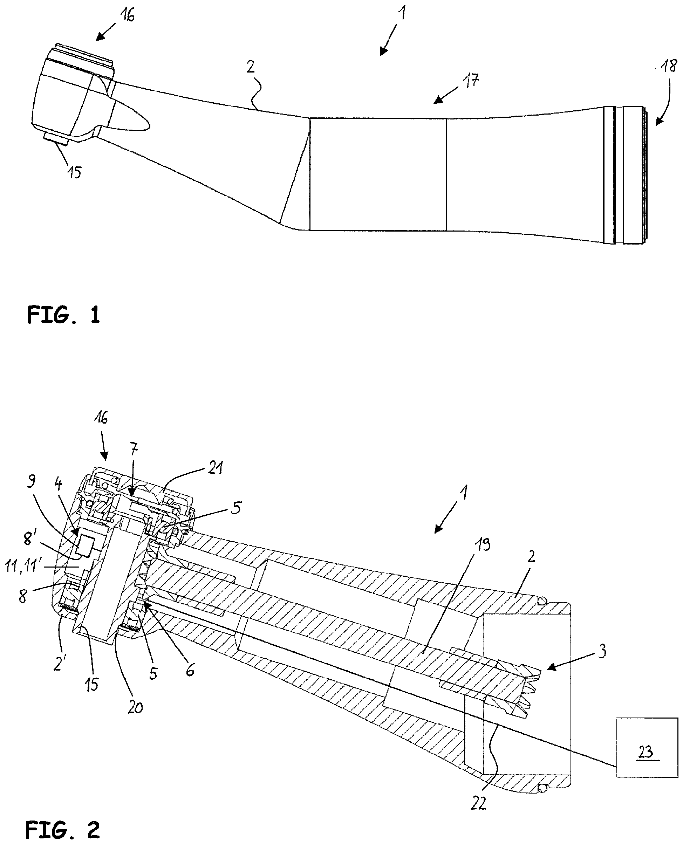

Die Figur 1 zeigt eine Außenansicht eines gebogenen Handstücks 1, welches oftmals als Winkelstück bezeichnet wird, mit seiner Außenhülse 2. Das Handstück 1 weist einen, insbesondere gebogenen, Griffteil 17 und einen Kopfteil 16 auf, in welchem ein Werkzeughalter 15 zum lösbaren Verbinden oder Aufnehmen eines mit dem Handstück 1 verbindbaren Werkzeugs angeordnet ist. Der Werkzeughalter 15 ist vorzugsweise beweglich im Handstück 1 angeordnet, zum Beispiel drehbar, wobei die Drehbarkeit insbesondere durch Lager 5, vorzugsweise Wälz- oder Kugellager, gewährleistet ist (siehe Figur 2).

An dem dem Kopfteil 16 gegenüber liegenden Ende des Handstücks 1 ist eine Kupplungs- oder Anschlussvorrichtung 18 vorgesehen, welche ausgebildet ist, das Handstück 1 mit einer oder mehreren Fluidquellen, zum Beispiel einer Flüssigkeitsquelle, insbesondere Wasser, oder einer Gasquelle, insbesondere Druckluft, zu verbinden. Vorzugsweise ist das Handstück 1 über die Anschlussvorrichtung 18 auch mit einer elektrischen Spannungsquelle und / oder einer Antriebsvorrichtung, zum Beispiel einen Elektromotor oder einem pneumatisch betreibbaren Motor, verbindbar. Wahlweise sind weitere elektrische Leitungen oder Kontakte zur Daten- und / oder Signalübertragung an der Kupplungsvorrichtung 18 vorgesehen.

Durch das Handstück 1 bzw. die Außenhülse 2 erstrecken sich eine oder mehrere Fluidleitungen von der Anschlussvorrichtung 18 in Richtung des Kopfteils 16 oder bis in den Kopfteil 16. Gegebenenfalls weist das Handstück 1 auch eine oder mehrere Wellen 19 auf, vorzugsweise mittels Zahnrädern oder Getrieben 6 verbunden, die als Antriebsvorrichtung 3 dienen und eine Antriebsbewegung auf den Werkzeughalter 15 übertragen (siehe Figur 2).

Die Figur 2 zeigt einen Schnitt durch einen Teil des Griffteils 17 und insbesondere durch den Kopfteil 16 des Handstücks 1. Der Kopfteil 16 weist eine hohle Kopfhülse 2' auf, die einen Innenraum 11 bildet. Der Innenraum 11 ist insbesondere als Fluid, insbesondere Luft, enthaltendes Volumen 11' ausgebildet, in dem unterschiedliche Bauteile des Kopfteils aufgenommen sind, zum Beispiel in Bezug auf die Hülse 2, 2' stationäre Bauteile oder in Bezug auf die Hülse 2, 2' bewegliche Bauteile, insbesondere die Lager 5, der Werkzeughalter 15, zumindest Teile eines Getriebes 6 oder von Zahnrädern, welche die Antriebsbewegung der Antriebsvorrichtung 3 von der Welle 19 auf den Werkzeughalter 15 übertragen, zumindest Teile einer Vorrichtung 7 zum Lösen des Werkzeugs aus dem Werkzeughalter oder weitere Bauteile wie Federelemente, Stützelemente, etc. An einer Seite des Kopfteils 16 ist des Weiteren eine Öffnung 20 vorgesehen, durch welche das Werkzeug in den Kopfteil 16 oder in den Werkzeughalter 15 einsetzbar ist bzw. daraus entnehmbar ist. An der Seite des Kopfteils 16, die der Öffnung 20 gegenüber liegt, ist ein Betätigungselement 21 zum Betätigen der Werkzeuglösevorrichtung 7 vorgesehen, insbesondere ein Druckelement oder Druckdeckel.

Zumindest einige der im Vorstehenden genannten Bauteile oder Teile davon können als Wärmequellen ausgebildet sein, zum Beispiel die Lager 5, das Getriebe 6 oder die Werkzeuglösevorrichtung 7. Selbstverständlich können jedoch auch andere Bauteile des Handstücks Wärmequellen bilden, insbesondere ein elektromagnetische Strahlung emittierendes Bauteil, zum Beispiel eine Beleuchtungsvorrichtung, ein Heizelement für Medien, ein elektrisches oder elektronisches Bauteil, etc. Gemeinsam ist allen diesen Wärmequellen, dass sie Wärme in das Innere des Handstücks 1 und / oder an die Außenhülse 2, 2' des Handstücks abgeben. Um die Temperatur des Handstücks 1 oder eines Teils des Handstücks 1, vorzugsweise der Außenhülse 2, 2', oder des Innenraums 11 des Handstücks 1 zu bestimmen und insbesondere eine zu starke Erwärmung zu verhindern, ist deshalb am oder im Handstück 1, insbesondere am oder im Kopfteil 16, eine Temperaturmessvorrichtung 4 vorgesehen.

Über eine elektrische Leitung oder eine Signalleitung 22 ist die Temperaturmessvorrichtung 4 mit einer Einheit 23 verbunden, die wahlweise als Auswerteeinheit zum Auswerten der Messsignale der Temperaturmessvorrichtung 4 und / oder als Regel- oder Steuereinheit zum temperaturabhängigen Regeln oder Steuern des Betriebs des Handstücks 1, zum Beispiel zum Unterbrechen des Betriebs des Handstücks 1 bei Erreichen oder Überschreiten eines vorbestimmten Temperaturgrenzwerts, und / oder als Anzeigeeinheit zum Anzeigen des gemessenen oder bestimmten Temperaturwerts oder eines Temperaturgrenzwerts ausgebildet ist. Vorzugsweise sind zumindest Teile der Einheit 23 am oder im Handstück 23 vorgesehen. Gemäß einem besonders bevorzugten Ausführungsbeispiel ist die gesamte Temperaturmessvorrichtung 4 einschließlich der Leitung 22 und der Einheit 23 im oder am Handstück 1 angeordnet, wodurch insbesondere ein in Bezug auf die Temperaturmessung, -auswertung und die davon abhängige Steuerung oder Regelung unabhängiges Handstück 1 entsteht.

Die im Vorstehenden unter Bezug auf die Figuren 1 und 2 beschriebenen Merkmale gelten für alle in den Figuren 2 - 13 beschriebenen Ausführungsbeispiele. Im Folgenden werden nun insbesondere jene Merkmale beschrieben, in denen sich die Handstücke 1 der Figuren 2 - 13 unterscheiden, bevorzugt die unterschiedlichen Temperaturmessvorrichtungen 4.

Die Handstücke 1 der Figur 2 - 4 weisen jeweils eine Temperaturmessvorrichtung 4 auf, die ausgebildet ist, die Temperatur des Handstücks 1 oder eines Teils des Handstücks 1 oder des durch die Wärmequellen 5, 6, 7 erwärmbaren Innenraums 11 berührungslos durch Detektion von elektromagnetischer Strahlung, insbesondere von Wärmestrahlung, zu messen.

Die Temperaturmessvorrichtungen 4 sind gemäß den Figuren 1 - 4 derart in den Handstücken 1 angeordnet und / oder ausgebildet, dass sie elektromagnetische Strahlung mehrerer in der Außenhülse 2 des Handstücks 1 angeordneter Wärmequellen (zum Beispiel der Lager 5, des Getriebes 6 und Teile der Werkzeuglösevorrichtung 7) empfangen, um insbesondere einen gemittelten Temperaturwert der mehreren Wärmequellen 5, 6, 7 zu bestimmen. Um die Wärmestrahlung der mehreren Wärmequellen 5, 6, 7 zu empfangen, ist zumindest eines der folgenden Merkmale verwirklicht: Die Temperaturmessvorrichtung 4 ist im Innenraum 11, 11' des Handstücks 1, beabstandet von den Wärmequellen 5, 6, 7 angeordnet; die Temperaturmessvorrichtung 4 ist zwischen den Wärmequellen 5, 6, 7 angeordnet; die Wärmequellen 5, 6, 7 umgeben die Temperaturmessvorrichtung 4; die elektromagnetische Strahlung emittierenden Oberflächen 8 (es ist in der Figur 2 beispielhaft nur eine derartige Oberfläche an einem Lager 5 dargestellt) der Wärmequellen 5, 6, 7 sind räumlich von einer die elektromagnetische Strahlung der Wärmequelle 5, 6, 7 empfangenden Oberfläche 8' der Temperaturmessvorrichtung 4 beabstandet; die Temperaturmessvorrichtung 4 weist mehrere elektromagnetische Strahlung der Wärmequelle 5, 6, 7 empfangende Oberflächen 8' auf, die insbesondere in unterschiedliche Richtungen weisen.

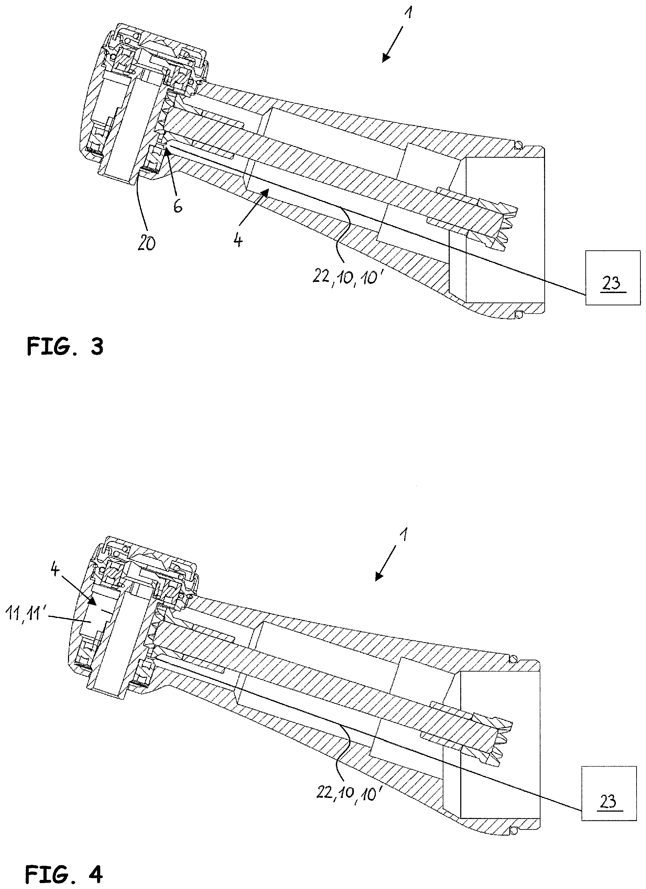

Vorzugsweise ist die Temperaturmessvorrichtung 4, insbesondere der Sensor der Temperaturmessvorrichtung 4, zwischen den beiden den Werkzeughalter 15 lagernden Lagern 5 und / oder in der Nähe der Kopfhülse 2', insbesondere an der Innenseite des sich zwischen dem Betätigungselement 21 und der Werkzeugöffnung 20 erstreckenden Mantels der Kopfhülse 2', und / oder in einem Raum zwischen der Kopfhülse 2' und dem Werkzeughalter 15 und / oder in einem Raum, der von der Kopfhülse 2, dem Werkzeughalter 15 und den beiden Lagern 5 begrenzt wird, angeordnet (siehe Figuren 2 und 4). Alternativ ist die Temperaturmessvorrichtung 4, insbesondere der Sensor der Temperaturmessvorrichtung 4, im Bereich der Werkzeuglösevorrichtung 7, insbesondere zwischen dem dem Betätigungselement 21 näher gelegenen Lager 5 und dem Betätigungselement 21 angeordnet. Alternativ ist die Temperaturmessvorrichtung 4, insbesondere der Sensor der Temperaturmessvorrichtung 4, in der Nähe jenes Lagers 5, das der Werkzeugöffnung 20 näher liegt, und / oder im Bereich des Getriebes 6 und / oder zwischen dem genannten Lager 5 und dem Getriebe 6 angeordnet (siehe Figur 3).

Die Temperaturmessvorrichtung 4 gemäß Figur 2 weist insbesondere ein Pyrometer oder einen Infrarotsensor 9 auf, die insbesondere ausgebildet sind, elektromagnetische Strahlung in einem Bereich von etwa 5 µm bis etwa 20 µm zu detektieren. Das Pyrometer oder der Infrarotsensor 9 sind bevorzugt als Halbleitersensor ausgebildet. Vorzugsweise umfasst das Pyrometer oder der Infrarotsensor 9 des Weiteren eine Vorrichtung zur Eigentemperaturkompensation, insbesondere einen Widerstand.

Die Temperaturmessvorrichtungen 4 gemäß den Figuren 3 und 4 weisen einen optischen Leiter 10 für elektromagnetische Strahlung auf, insbesondere einen Faserstab 10', vorzugsweise aus Quarzglas, dessen Streuung oder Brechungsindex, insbesondere im temperatursensitive Abschnitt, aufgrund von Temperaturänderungen variiert. In der Einheit 23 ist eine elektromagnetische Strahlungsquelle vorgesehen, welche Strahlung in den optischen Leiter abgibt. Diese Strahlung wird durch den optischen Leiter geleitet, in Abhängigkeit der Temperatur des Handstücks 1 oder des Innenraums 11 und damit in Abhängigkeit der Streuung oder des Brechungsindexes des optischen Leiters 10 gestreut bzw. gebrochen und kehrt anschließend zur Einheit 23 zurück. Die Einheit 23 ermittelt auf Basis der Veränderungen der empfangenen Strahlung in Bezug auf die von der Strahlungsquelle emittierte Strahlung die Temperatur (faseroptische Temperaturmessung oder distributed temperature sensing).

Wie im Vorstehenden bereits beschrieben, ist das Ende des optischen Leiters 10 oder der temperatursensitive Abschnitt des optischen Leiters 10 entweder im Bereich des der Werkzeugöffnung 20 näher gelegenen Lagers 5 (siehe Figur 3) oder in einem Raum zwischen der Kopfhülse 2' und dem Werkzeughalter 15 angeordnet (siehe Figur 4).

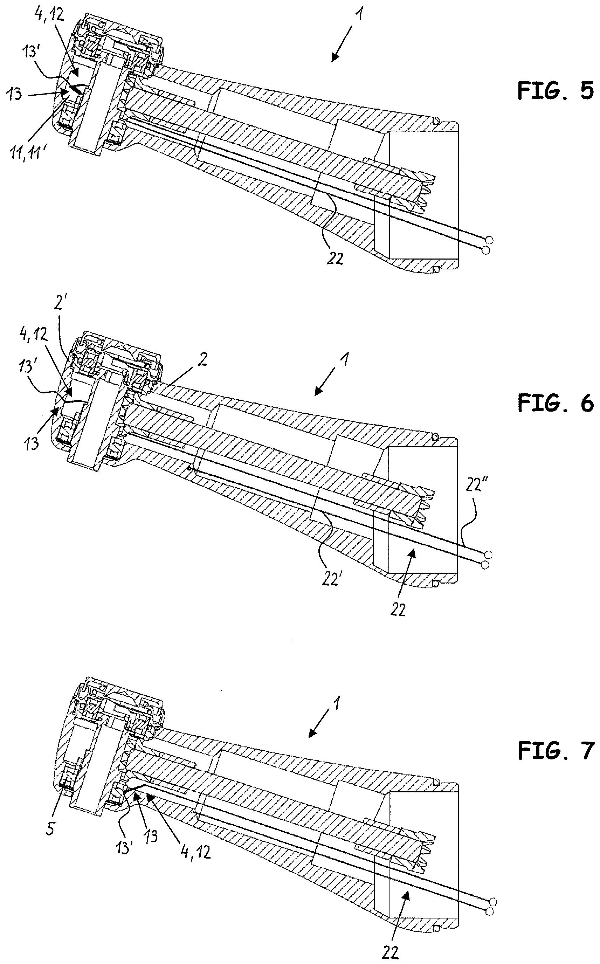

Die Figuren 5 - 10 zeigen unterschiedliche Ausführungsbeispiele eines Handstücks 1 mit einer elektrischen Temperaturmessvorrichtung 12, die insbesondere zumindest ein Thermoelement 13 oder eine Thermoelementverbindung 13' aufweist, welche ausgebildet sind, zur Messung der Temperatur ein elektrisches Thermospannungssignal zur Verfügung zu stellen. Das Thermoelement 13 oder die Thermoelementverbindung 13' weisen zwei unterschiedliche Materialien auf, insbesondere zwei unterschiedliche Metalle, die an einem Punkt oder Abschnitt miteinander verbunden sind, insbesondere verschweißt. Eine elektrische Leitung 22 leitet das Thermospannungssignal oder Messsignal des Thermoelements 13 zu der Einheit 23, die auf Basis des empfangenen Signals den Temperaturwert ermittelt.

Das Thermoelement 13 oder die Thermoelementverbindung 13' der Figur 5 ist ausgebildet und / oder angeordnet, die Temperatur des durch die zumindest eine Wärmequelle 5, 6, 7 erwärmbaren Innenraums 11 des Handstücks 1, bevorzugt die Temperatur des im Innenraum 11 oder Volumen 11' enthaltenen Fluids, zu messen. Alternativ ist das Thermoelement 13 oder die Thermoelementverbindung 13' der Figur 5 derart ausgebildet und / oder angeordnet, dass sie durch die Messung der Temperatur des Innenraums 11, 11' einen gemittelten Temperaturwert oder Durchschnittstemperaturwert ermittelt, insbesondere einen gemittelten Temperaturwert oder Durchschnittstemperaturwert mehrerer Bauteile und / oder Wärmequellen 5, 6, 7 des Handstücks 1, welche Wärme in den Innenraum 11, 11' oder an das darin enthaltene Fluid abgeben.

Um die Temperatur des Innenraums 11, 11' ermitteln zu können, ist zumindest eines der folgenden Merkmale verwirklicht: Das Thermoelement 13 oder die Thermoelementverbindung 13' ist im Innenraum 11, 11' des Handstücks 1, beabstandet von den Wärmequellen 5, 6, 7 angeordnet; das Thermoelement 13 oder die Thermoelementverbindung 13' ist zwischen den Wärmequellen 5, 6, 7 angeordnet; die Wärmequellen 5, 6, 7 umgeben das Thermoelement 13 oder die Thermoelementverbindung 13'; die Wärme emittierenden Oberflächen 8 der Wärmequellen 5, 6, 7 sind räumlich von dem Thermoelement 13 oder der Thermoelementverbindung 13' beabstandet.

Die Figur 5 zeigt konkret ein Thermoelement 13, das zwischen den beiden den Werkzeughalter 15 lagernden Lagern 5 und / oder in der Nähe der Kopfhülse 2', insbesondere an der Innenseite des sich zwischen dem Betätigungselement 21 und der Werkzeugöffnung 20 erstreckenden Mantels der Kopfhülse 2', und / oder in einem Raum zwischen der Kopfhülse 2' und dem Werkzeughalter 15 und / oder in einem Raum, der von der Kopfhülse 2, dem Werkzeughalter 15 und den beiden Lagern 5 begrenzt wird, angeordnet ist.

Alterativ sind in den Figuren 6 und 7 zwei Ausführungsbeispiele dargestellt, bei denen das Thermoelement 13 oder die Thermoelementverbindung 13' jeweils an einem Bauteil des Handstücks 1, nämlich der Außenhülse 2 (siehe Figur 5) oder einem Lager 5 (siehe Figur 6) vorgesehen ist, so dass das Thermoelement 13 oder die Thermoelementverbindung 13' vorzugsweise die Temperatur dieses Bauteils ermittelt. Erfindungsgemäß weist das Bauteil 2, 5, an dem das Thermoelement 13 oder die Thermoelementverbindung 13' vorgesehen ist, ein Material auf, welches Teil des Thermoelements 13 oder der Thermoelementverbindung 13' ist.

Ein Vorteil der in den Figuren 6 und 7 dargestellten Ausführungsbeispiele liegt insbesondere darin, dass aufgrund der Verwendung eines Materials der Außenhülse 2, 2' als Teil des Thermoelements 13 oder der Thermoelementverbindung 13' oder aufgrund der elektrischen Verbindung der Außenhülse 2, 2' mit dem Thermoelement 13 oder der Thermoelementverbindung 13' oder aufgrund der elektrischen Verbindung der Außenhülse 2, 2' mit dem Bauteil 2, 5, dessen Material Teil des Thermoelements 13 oder der Thermoelementverbindung 13' ist, zumindest ein elektrischer Leiter 22' der elektrischen Leitung 22, die das Thermospannungssignal zur Einheit 23 leitet, das Thermospannungssignal nicht direkt an dem Thermoelement 13 oder der Thermoelementverbindung 13' abnehmen muss, sondern dass das Thermospannungssignal an (einer beliebigen Stelle) der Außenhülse 2, 2' abnehmbar ist.

In anderen Worten umfasst die Temperaturvorrichtung 4 gemäß den Figuren 6 und 7 ein Thermoelement 13 oder eine Thermoelementverbindung 13' und eine elektrische Leitung 22 mit zwei elektrischen Leitern 22', 22" zum Leiten des von dem Thermoelement 13 oder der Thermoelementverbindung 13' erzeugten Thermospannungssignals an die Auswerteeinheit 23, wobei das Thermoelement 13 oder die Thermoelementverbindung 13' an der Außenhülse 2, 2' oder an einem mit der Außenhülse 2, 2' (elektrisch) verbundenen Bauteil, zum Beispiel Lager 5, vorgesehen ist (um die Temperatur der Außenhülse 2, 2' oder des damit verbundenen Bauteils zu bestimmen), wobei die Außenhülse 2, 2' oder das Bauteil ein Material aufweist, welches Teil des Thermoelements 13 oder der Thermoelementverbindung 13' ist und wobei einer der beiden Leiter 22', 22" die Außenhülse 2, 2' des Handstücks 1 (elektrisch) kontaktiert, insbesondere eine beliebige Stelle der Außenhülse 2, 2' kontaktiert, vorzugsweise eine von dem Thermoelement 13 oder der Thermoelementverbindung 13' beabstandete Stelle der Außenhülse 2, 2', zum Beispiel den Griffteil 17 der Außenhülse 2 oder einen an den Kopfteil 16 anschließenden Abschnitt der Außenhülse 2 oder die Kupplungs- oder Anschlussvorrichtung 18 oder einen zwischen dem Thermoelement 13 oder der Thermoelementverbindung 13' und der Kupplungs- oder Anschlussvorrichtung 18 angeordnete Stelle der Außenhülse 2.

Alternativ ist es selbstverständlich in entsprechender Weise möglich, dass einer der beiden Leiter 22', 22" das Bauteil, dessen Material Teil des Thermoelements 13 oder der Thermoelementverbindung 13' ist, zum Beispiel das Lager 5, (elektrisch) kontaktiert, insbesondere eine beliebige Stelle des Bauteils kontaktiert. In diesem Fall ist klarerweise eine elektrische Verbindung des Bauteils zur Außenhülse 2, 2' nicht notwendig.

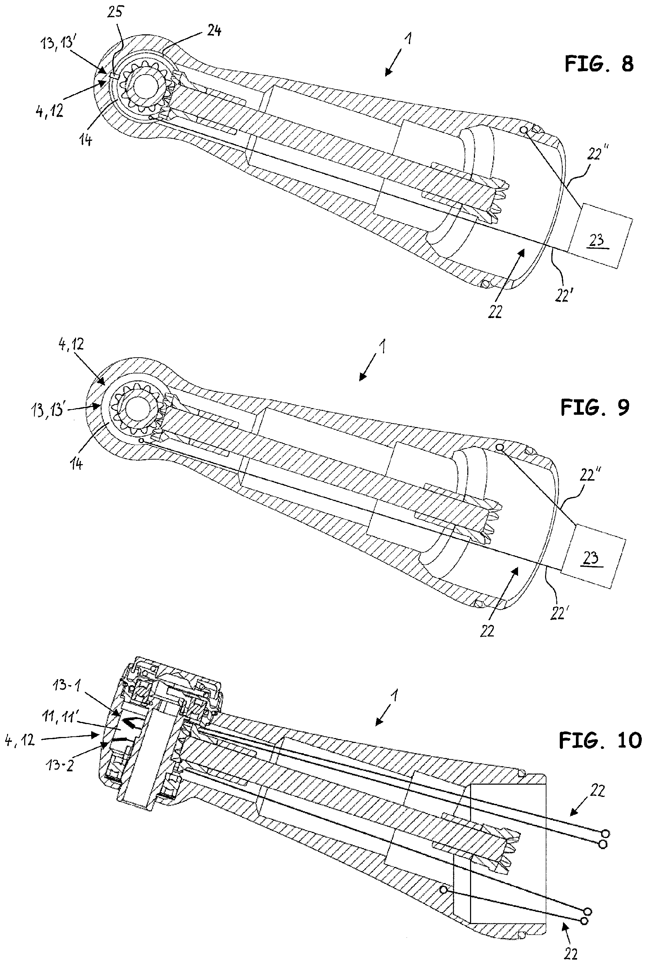

Die Figuren 8 und 9 zeigen zwei weitere Ausführungsbeispiele eines Handstücks 1 mit einem Thermoelement 13 oder einer Thermoelementverbindung 13', wobei bei beiden Ausführungsbeispielen ein, insbesondere metallischer, Kontaktring 14 vorgesehen ist, der Teil des Thermoelements 13 ist oder der einen Teil der Thermoelementverbindung 13' bildet.

In der Figur 8 ist der Kontaktring 14 durch eine elektrische Isolierung 24, zum Beispiel einer ringförmigen Isolierung, vom Handstück getrennt. Die elektrische Isolierung 24 ist insbesondere zwischen der Außenhülse 2, 2' des Handstücks 1 und dem Kontaktring 14 vorgesehen. An einer (räumlich begrenzten, schmalen oder punktförmigen) Kontaktstelle oder einem Kontaktpunkt 25 ist die Isolierung 24 unterbrochen oder weist einen Durchbruch auf, so dass ein direkter elektrischer Kontakt zwischen dem Kontaktring 14, der ein erstes Material aufweist, und der Außenhülse 2, 2' des Handstücks 1, die ein zweites, unterschiedliches Material aufweist, gebildet ist. Dieser die elektrische Isolierung 24 durchsetzende Kontaktpunkt 25 oder die beiden Materialien der Außenhülse 2, 2' und des Kontaktrings 14 bilden somit das Thermoelement 13 oder die Thermoelementverbindung 13' zur Messung der Temperatur. Das von dem Thermoelement 13 oder der Thermoelementverbindung 13' zur Verfügung gestellte Thermospannungssignal repräsentiert somit einen Temperaturwert an dem Punktkontakt zwischen der Außenhülse 2, 2' und dem Kontaktring 14. Vorzugsweise ist der Kontaktring 14 im Bereich des Betätigungselements 21 zum Betätigen der Werkzeuglösevorrichtung 7 vorgesehen, insbesondere zwischen dem Betätigungselement 21 und dem Lager 5, das dem Betätigungselement 21 näher liegt.

Das Handstück 1 der Figur 9 weist im Wesentlichen denselben Aufbau wie das Handstück 1 der Figur 8 auf, wobei jedoch keine elektrische Isolierung 24 zwischen der Außenhülse 2, 2' und dem Kontaktring 14 vorgesehen ist, so dass hier - anstelle des Punktkontakts der Figur 8 - zwischen der Außenhülse 2, 2' und dem Kontaktring 14 bzw. deren unterschiedlichen Materialien ein elektrischer Flächenkontakt vorliegt, der die Thermoelementverbindung 13' bildet. Der Flächenkontakt umfasst beispielhaft einen Winkel von zumindest 5° der Umfangsfläche des Kontaktrings 14, bevorzugt von zumindest 90°, insbesondere von mehr als 180°. Die von dem Thermoelement 13 oder der Thermoelementverbindung 13' zur Verfügung gestellte Thermospannung repräsentiert somit einen gemittelten Temperaturwert oder einen Durchschnittstemperaturwert an dem oder über den Flächenkontakt zwischen der Außenhülse 2, 2' und dem Kontaktring 14.

In den Handstücken 1 der Figuren 8 und 9 ist ebenfalls jeweils eine elektrische Leitung 22 zur Leitung des Thermospannungssignals vorgesehen, wobei einer der beiden Leiter 22' den Kontaktring 14 und der andere der beiden Leiter 22" die Außenhülse 2, 2' kontaktiert. Wie unter Bezug auf die Figuren 6 und 7 erläutert, kann der Leiter 22" die Außenhülse 2, 2' wiederum an einer beliebigen Stelle kontaktieren, vorzugsweise eine von dem Thermoelement 13 oder der Thermoelementverbindung 13' oder dem Kontaktring 14 beabstandete Stelle der Außenhülse 2, 2', zum Beispiel den Griffteil 17 der Außenhülse 2 oder einen an den Kopfteil 16 anschließenden Abschnitt der Außenhülse 2 oder die Kupplungs- oder Anschlussvorrichtung 18 oder einen zwischen dem Thermoelement 13 oder der Thermoelementverbindung 13' und der Kupplungs- oder Anschlussvorrichtung 18 angeordnete Stelle der Außenhülse 2.

Selbstverständlich ist es auch möglich, in einem Handstück 1 mehrere Thermoelemente 13 oder Thermoelementverbindungen 13' vorzusehen, so wie dies beispielhaft in der Figur 10 dargestellt ist. Das Handstück 1 der Figur 10 weist ein erstes Thermoelement 13-1 auf, das frei, ohne direkten Kontakt zu einem Bauteil des Handstücks 1 im Innenraum 11, 11' vorgesehen ist, um insbesondere die Temperatur des im Innenraum 11, 11' enthaltenen Fluids zu messen, so wie es im Vorstehenden für das Handstück 1 der Figur 5 beschrieben ist. Demgemäß sind alle in Zusammenhang mit dem Handstück 1 oder dem Thermoelement 13 der Figur 5 beschriebenen Merkmale in entsprechender Weise auf das Handstück 1 der Figur 10 oder das Thermoelement 13-1 anwendbar oder übertragbar.

Das Handstück 1 der Figur 10 weist des Weiteren ein zweites Thermoelement 13-2 auf, das an einem Bauteil des Handstücks 1 vorgesehen ist. Insbesondere weist das Bauteil ein Material auf, das Teil des Thermoelements 13-2 ist. Vorzugsweise ist das Bauteil durch die Außenhülse 2, 2' gebildet, so wie es im Vorstehenden für das Handstück 1 der Figur 6 beschrieben ist. Demgemäß sind alle in Zusammenhang mit dem Handstück 1 oder dem Thermoelement 13 der Figur 6 beschriebenen Merkmale in entsprechender Weise auf das Handstück 1 der Figur 10 oder das Thermoelement 13-2 anwendbar oder übertragbar.

Von jedem Thermoelement 13-1, 13-2 des Handstücks 1 führt eine elektrische Leitung 22 zu der Einheit 23, welche die Thermospannungssignale jedes Thermoelements 13-1, 13-2 empfängt und verarbeitet.

Weist ein Handstück 1 mehrere Thermoelement 13 oder der Thermoelementverbindung 13' auf, so sind selbstverständlich ihre Anzahl, als auch ihre Position im Handstück 1 oder ihre Bauweise je nach Anforderungen oder Wunsch, wo die Temperatur gemessen werden soll, frei wählbar. So können zum Beispiel in einem Handstück 1 mehrere freie Thermoelement 13-1 ohne direkten Kontakt zu einem Bauteil des Handstücks 1 und / oder mehrere an einem Bauteil und / oder an mehreren unterschiedlichen Bauteilen des Handstücks 1 angeordnete Thermoelement 13-2 vorgesehen sein.

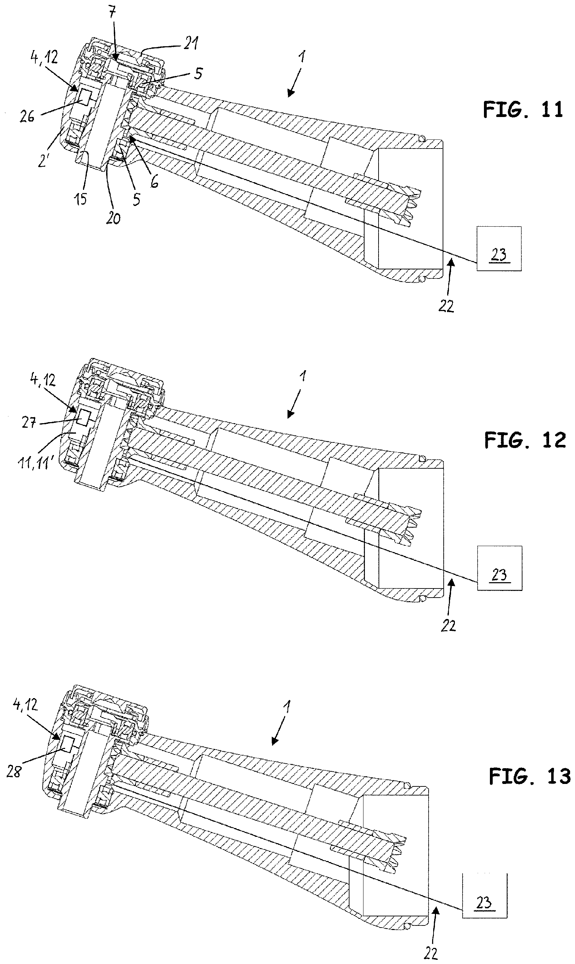

Die Figuren 11 - 13 zeigen Handstücke 1 mit weiteren elektrischen Temperaturmessvorrichtungen 12, wobei jede dieser Temperaturmessvorrichtungen 12 ein Material umfasst, dessen elektrischer Widerstand oder dynamischer Widerstand in Abhängigkeit der Temperatur variabel ist.

Die Temperaturmessvorrichtungen 4, 12 der Figuren 11 - 13 sind vorzugsweise derart in den Handstücken 1 angeordnet und / oder ausgebildet, dass sie Wärme oder Wärmestrahlung mehrerer in der Außenhülse 2 des Handstücks 1 angeordneter Wärmequellen (zum Beispiel der Lager 5, des Getriebes 6 und Teile der Werkzeuglösevorrichtung 7) empfangen, um insbesondere einen gemittelten Temperaturwert der mehreren Wärmequellen 5, 6, 7 zu bestimmen. Um die Wärme oder Wärmestrahlung der mehreren Wärmequellen 5, 6, 7 zu empfangen, ist zumindest eines der folgenden Merkmale verwirklicht: Die Temperaturmessvorrichtung 4, 12 ist im Innenraum 11, 11' des Handstücks 1, beabstandet von den Wärmequellen 5, 6, 7 angeordnet; die Temperaturmessvorrichtung 4, 12 ist zwischen den Wärmequellen 5, 6, 7 angeordnet; die Wärmequellen 5, 6, 7 umgeben die Temperaturmessvorrichtung 4, 12; die Wärme emittierenden Oberflächen der Wärmequellen 5, 6, 7 sind räumlich von einer die Wärme der Wärmequelle 5, 6, 7 empfangenden Oberfläche der Temperaturmessvorrichtung 4, 12 beabstandet; die Temperaturmessvorrichtung 4, 12 weist mehrere Wärme der Wärmequelle 5, 6, 7 empfangende Oberflächen 8' auf, die insbesondere in unterschiedliche Richtungen weisen.

Alternativ sind die Temperaturmessvorrichtungen 4, 12 der Figuren 11 - 13 ausgebildet und / oder angeordnet, die Temperatur des durch die zumindest eine Wärmequelle 5, 6, 7 erwärmbaren Innenraums 11 des Handstücks 1, bevorzugt die Temperatur des im Innenraum 11 oder Volumen 11' enthaltenen Fluids, zu messen.

Vorzugsweise ist die Temperaturmessvorrichtung 4, 12, insbesondere der Sensor der Temperaturmessvorrichtung 4, 12, zwischen den beiden den Werkzeughalter 15 lagernden Lagern 5 und / oder in der Nähe der Kopfhülse 2', insbesondere an der Innenseite des sich zwischen dem Betätigungselement 21 und der Werkzeugöffnung 20 erstreckenden Mantels der Kopfhülse 2', und / oder in einem Raum zwischen der Kopfhülse 2' und dem Werkzeughalter 15 und / oder in einem Raum, der von der Kopfhülse 2, dem Werkzeughalter 15 und den beiden Lagern 5 begrenzt wird, angeordnet (siehe Figuren 2 und 4). Alternativ ist die Temperaturmessvorrichtung 4, 12, insbesondere der Sensor der Temperaturmessvorrichtung 4, 12, im Bereich der Werkzeuglösevorrichtung 7, insbesondere zwischen dem dem Betätigungselement 21 näher gelegenen Lager 5 und dem Betätigungselement 21 angeordnet. Alternativ ist die Temperaturmessvorrichtung 4, 12, insbesondere der Sensor der Temperaturmessvorrichtung 4, 12, in der Nähe jenes Lagers 5, das der Werkzeugöffnung 20 näher liegt, angeordnet.

Die Temperaturmessvorrichtung 4, 12 des Handstücks 1 der Figur 11 weist einen Messwiderstand 26 auf, der vorzugsweise aus einem Edelmetall besteht, dessen elektrischer Widerstand (ohmsche Widerstand) in Abhängigkeit der Temperatur, insbesondere der Temperatur des den Widerstand 26 umgebenden Fluids des Innenraums 11, 11', variabel ist. Eine in der Einheit 23 vorgesehene Strom- oder Spannungsquelle bildet mit der elektrischen Leitung 22 und dem Messwiderstand 26 einen elektrischen Stromkreis. Die Veränderung des von der Strom- oder Spannungsquelle abgegebenen, über den Messwiderstand 26 geleiteten elektrischen Messsignals, insbesondere der Spannungsabfall eines als Messsignal dienenden konstanten Messstroms, wird von einem Schaltkreis der Einheit 23 detektiert und zur Bestimmung der Temperatur verwendet. Vorzugsweise sind in der Einheit weitere Elemente zur Verarbeitung des Messsignals vorhanden, zum Beispiel Analog-Digital-Wandler, Signalverstärker oder Filter.

Die Temperaturmessvorrichtung 4, 12 des Handstücks 1 der Figur 12 weist einen Halbleitersensor 27 auf, vorzugsweise eine Halbleiterdiode, insbesondere eine dotierte Siliziumdiode, dessen dynamischer Widerstand (Schleusenspannung an einem p-n-Übergang oder einem Metall-Halbleiter-Übergang des Halbleitersensors 27) in Abhängigkeit der Temperatur, insbesondere der Temperatur des den Halbleitersensor 27 umgebenden Fluids des Innenraums 11, 11', variabel ist. Eine in der Einheit 23 vorgesehene Strom- oder Spannungsquelle bildet mit der elektrischen Leitung 22 und dem Halbleitersensor 27 einen elektrischen Stromkreis. Die Veränderung des von der Strom- oder Spannungsquelle abgegebenen, über den Halbleitersensor 27 geleiteten elektrischen Messsignals, insbesondere der Spannungsabfall eines als Messsignal dienenden konstanten Messstroms, wird von einem Schaltkreis der Einheit 23 detektiert und zur Bestimmung der Temperatur verwendet. Vorzugsweise sind in der Einheit weitere Elemente zur Verarbeitung des Messsignals vorhanden, zum Beispiel Analog-Digital-Wandler, Signalverstärker oder Filter.

Die Temperaturmessvorrichtung 4, 12 des Handstücks 1 der Figur 12 weist einen metallischen Widerstand 28 auf, wobei die Spannung des thermischen Widerstandsrauschens (d.h. die Dichteschwankungen der Transportelektronen des Widerstands 28 aufgrund ihrer thermischen Bewegung) in Abhängigkeit der Temperatur, insbesondere der Temperatur des den Widerstand 28 umgebenden Fluids des Innenraums 11, 11', variabel ist. Eine in der Einheit 23 vorgesehene Strom- oder Spannungsquelle bildet mit der elektrischen Leitung 22 und dem Widerstand 28 einen elektrischen Stromkreis. Die Veränderung des von der Strom- oder Spannungsquelle abgegebenen, über den Widerstand 28 geleiteten elektrischen Messsignals, insbesondere die Veränderung der elektrischen Spannung, wird von einem Schaltkreis der Einheit 23 detektiert und zur Bestimmung der Temperatur verwendet. Vorzugsweise sind in der Einheit weitere Elemente zur Verarbeitung des Messsignals vorhanden, zum Beispiel Analog-Digital-Wandler, Signalverstärker oder Filter.

Die Erfindung ist nicht auf die beschriebenen Ausführungsbeispiele beschränkt, sondern umfasst alle Ausführungen, die das prinzipielle, sinngemäße Funktionsprinzip der in den Ansprüchen 1 bis 13 definierten Erfindung anwenden oder beinhalten. So ist insbesondere die Position der Temperaturvorrichtung 4 im Handstück 1 variabel und nicht ausschließlich auf den Kopfteil 16 beschränkt. Generell kann die Temperaturvorrichtung 4 an beliebigen Stellen im Handstück 1 angeordnet sein, insbesondere dort, wo Wärmequellen im Handstück 1 positioniert sind oder wo eine Außenfläche des Handstücks 1 besonders warm wird.

Des Weiteren sind alle Merkmale aller beschriebenen und dargestellten Ausführungsbeispiele miteinander kombinierbar. So ist es zum Beispiel auch möglich, unterschiedliche Sensoren in einem Handstück 1 vorzusehen, zum Beispiel einen Infrarotsensor 9 und ein Thermoelement 13. Die von den unterschiedlichen Sensoren abgegebenen Messsignale können entweder getrennt voneinander ausgewertet werden und / oder für eine gemeinsame, kombinierte Auswertung zusammengeführt werden, zum Beispiel zur Bildung eines Temperatur-Mittelwerts oder eines gewichteten Temperaturwerts, vorzugsweise durch eine Steuer-, Regel- und / oder Versorgungseinheit 23.

## Patentansprüche

1. Medizinisches, insbesondere dentales, Handstück (1), umfassend: eine Außenhülse (2), eine Antriebsvorrichtung (3) zum in Bewegung Versetzen eines mit dem Handstück (1) verbindbaren Werkzeugs und eine Temperaturmessvorrichtung (4) zur Messung der Temperatur des Handstücks (1) oder eines Teils des Handstücks (1), welche eine elektrische Temperaturmessvorrichtung (12) umfasst, die zumindest ein Thermoelement (13) aufweist, welches ausgebildet ist, zur Messung der Temperatur ein elektrisches Thermospannungssignal zur Verfügung zu stellen, wobei das zumindest eine Thermoelement (13) an einem Bauteil des Handstücks (1) vorgesehen ist, das ein Material aufweist, welches Teil des Thermoelements (13) ist, **dadurch gekennzeichnet, dass**
das Bauteil, dessen Material Teil des Thermoelements (13) ist, durch die Außenhülse (2) oder ein mit der Außenhülse (2) elektrisch leitend verbundenes Bauteil des Handstücks (1) gebildet ist.

2. Medizinisches, insbesondere dentales, Handstück (1), umfassend: eine Außenhülse (2), eine Antriebsvorrichtung (3) zum in Bewegung Versetzen eines mit dem Handstück (1) verbindbaren Werkzeugs und eine Temperaturmessvorrichtung (4) zur Messung der Temperatur des Handstücks (1) oder eines Teils des Handstücks (1), welche eine elektrische Temperaturmessvorrichtung (12) umfasst, die zumindest ein Thermoelement (13) aufweist, welches ausgebildet ist, zur Messung der Temperatur ein elektrisches Thermospannungssignal zur Verfügung zu stellen, wobei das zumindest eine Thermoelement (13) an einem Bauteil des Handstücks (1) vorgesehen ist, das ein Material aufweist, welches Teil des Thermoelements (13) ist, **dadurch gekennzeichnet, dass**
das Bauteil, dessen Material Teil des Thermoelements (13) ist, durch ein Lager (5) des Handstücks (1) gebildet ist.

3. Medizinisches, insbesondere dentales, Handstück (1), umfassend: eine Außenhülse (2), eine Antriebsvorrichtung (3) zum in Bewegung Versetzen eines mit dem Handstück (1) verbindbaren Werkzeugs und eine Temperaturmessvorrichtung (4) zur Messung der Temperatur des Handstücks (1) oder eines Teils des Handstücks (1), welche eine elektrische Temperaturmessvorrichtung (12) umfasst, die zumindest ein Thermoelement (13) aufweist, welches ausgebildet ist, zur Messung der Temperatur ein elektrisches Thermospannungssignal zur Verfügung zu stellen, wobei das zumindest eine Thermoelement (13) an einem Bauteil des Handstücks (1) vorgesehen ist, das ein Material aufweist, welches Teil des Thermoelements (13) ist, **dadurch gekennzeichnet, dass**
das Bauteil, dessen Material Teil des Thermoelements (13) ist, durch einen mit der Außenhülse (2) elektrisch leitend verbundenen Kontaktring (14) gebildet ist, wobei der Kontaktring (14) insbesondere an einem Betätigungselement (21) zum Betätigen der Werkzeuglösevorrichtung (7) vorgesehen ist.

4. Medizinisches, insbesondere dentales, Handstück (1), nach einem der vorstehenden Ansprüche, **dadurch gekennzeichnet, dass**
das Handstück (1) einen Handstückkopf (16) aufweist, in welchem ein Werkzeughalter (15) für das mit dem Handstück (1) verbindbare Werkzeug und das zumindest eine Thermoelement (13) vorgesehen sind.

5. Medizinisches, insbesondere dentales, Handstück (1), nach Anspruch 2 oder Anspruch 3, **dadurch gekennzeichnet, dass** das Handstück (1) einen Handstückkopf (16) aufweist und das Lager (5) oder der Kontaktring (14) in dem Handstückkopf (16) angeordnet ist.

6. Medizinisches, insbesondere dentales, Handstück (1), nach einem der vorstehenden Ansprüche, **dadurch gekennzeichnet, dass**
das zumindest eine Thermoelement (13) in einem Innenraum (11) des Handstücks (1) angeordnet ist, wobei der Innenraum (11) durch ein Fluid, insbesondere Luft, enthaltendes Volumen (11') gebildet ist, welches Bauteile des Handstücks (1) voneinander trennt.

7. Medizinisches, insbesondere dentales, Handstück (1), nach einem der vorstehenden Ansprüche, **gekennzeichnet durch**
eine elektrische Leitung (22), welche das Thermospannungssignal oder Messsignal des zumindest einen Thermoelements (13) zu einer Einheit (23) leitet, die auf Basis des empfangenen Signals den Temperaturwert ermittelt.

8. Medizinisches, insbesondere dentales, Handstück (1), nach Anspruch 7, **dadurch gekennzeichnet, dass**
die elektrische Leitung (22) einen ersten und zweiten elektrischen Leiter (22', 22") zum Leiten des Thermospannungssignals oder Messsignals des zumindest einen Thermoelements (13) zu der Einheit (23) aufweist, wobei zumindest einer der elektrischen Leiter (22') über die Außenhülse (2) mit dem Thermoelement (13) verbunden ist, so dass das Thermospannungssignal oder Messsignal von dem Thermoelement (13) über die Außenhülse (2) und den elektrischen Leiter (22') zu der Einheit (23) übertragbar ist.

9. Medizinisches, insbesondere dentales, Handstück (1), nach Anspruch 7, **dadurch gekennzeichnet, dass**
die elektrische Leitung (22) einen ersten und zweiten elektrischen Leiter (22', 22") zum Leiten des Thermospannungssignals oder Messsignals des zumindest einen Thermoelements (13) zu der Einheit (23) aufweist, wobei einer der Leiter (22', 22") das Bauteil, dessen Material Teil des Thermoelements (13) ist, zum Beispiel das Lager (5), kontaktiert.

10. Medizinisches, insbesondere dentales, Handstück (1), nach einem der vorstehenden Ansprüche 3 - 9, **dadurch gekennzeichnet, dass**
der Kontaktring (14) durch eine elektrische Isolierung (24) vom Handstück (1), insbesondere der Außenhülse (2), getrennt ist, wobei an einer Kontaktstelle oder einem Kontaktpunkt (25) die elektrische Isolierung (24) unterbrochen ist, so dass ein direkter elektrischer Kontakt zwischen dem Kontaktring (14), der ein erstes Material aufweist, und der Außenhülse (2), die ein zweites, unterschiedliches Material aufweist, gebildet ist, wobei der direkte elektrische Kontakt des ersten und zweiten Materials das Thermoelement (13) zur Messung der Temperatur bildet.

11. Medizinisches, insbesondere dentales, Handstück (1), nach einem der vorstehenden Ansprüche, **dadurch gekennzeichnet, dass**
in dem Handstück (1) zumindest ein weiteres Thermoelement (13) zur Messung der Temperatur des Handstücks (1) oder eines Teils des Handstücks (1) vorgesehen ist.

12. Medizinisches, insbesondere dentales, Handstück (1), nach einem der vorstehenden Ansprüche, **dadurch gekennzeichnet, dass**
in dem Handstück (1) zumindest eine weitere elektrische Temperaturmessvorrichtung (12) vorgesehen ist, insbesondere eine elektrische Temperaturmessvorrichtung (12) mit einem Material, dessen elektrischer Widerstand oder dynamischer Widerstand in Abhängigkeit der Temperatur variabel ist.

13. Medizinisches, insbesondere dentales, Handstück (1), nach einem der Ansprüche 7 - 12, **dadurch gekennzeichnet, dass**
die Einheit (23) als Auswerteeinheit zum Auswerten der Messsignale der Temperaturmessvorrichtung (4) und / oder als Regel- oder Steuereinheit zum temperaturabhängigen Regeln oder Steuern des Betriebs des Handstücks (1), zum Beispiel zum Unterbrechen des Betriebs des Handstücks (1) bei Erreichen oder Überschreiten eines vorbestimmten Temperaturgrenzwerts, und / oder als Anzeigeeinheit zum Anzeigen des gemessenen oder bestimmten Temperaturwerts oder eines Temperaturgrenzwerts ausgebildet ist.

## Claims

1. Medical, in particular dental handpiece (1), comprising: an outer sleeve (2), a drive device (3) for setting in motion a tool which is connectible to the handpiece (1) and a temperature measuring device (4) for measuring the temperature of the handpiece (1) or of a part of the handpiece (1), comprising an electrical temperature measuring device (12) having at least one thermocouple (13), which is configured to provide a thermoelectric voltage signal for the measurement of the temperature, wherein the at least one thermocouple (13) is provided on a component of the handpiece (1) that comprises a material which is part of the thermocouple (13), **characterized in that** the component whose material is part of the thermocouple (13) is formed by the outer sleeve (2) or a component of the handpiece (1) which is connected to the outer sleeve (2) in an electrically conductive manner.

2. Medical, in particular dental handpiece (1), comprising: an outer sleeve (2), a drive device (3) for setting in motion a tool which is connectible to the handpiece (1) and a temperature measuring device (4) for measuring the temperature of the handpiece (1) or of a part of the handpiece (1), comprising an electrical temperature measuring device (12) having at least one thermocouple (13), which is configured to provide a thermoelectric voltage signal for the measurement of the temperature, wherein the at least one thermocouple (13) is provided on a component of the handpiece (1) that comprises a material which is part of the thermocouple (13), **characterized in that** the component whose material is part of the thermocouple (13) is formed by a bearing (5) of the handpiece (1).

3. Medical, in particular dental handpiece (1), comprising: an outer sleeve (2), a drive device (3) for setting in motion a tool which is connectible to the handpiece (1) and a temperature measuring device (4) for measuring the temperature of the handpiece (1) or of a part of the handpiece (1), comprising an electrical temperature measuring device (12) having at least one thermocouple (13), which is configured to provide a thermoelectric voltage signal for the measurement of the temperature, wherein the at least one thermocouple (13) is provided on a component of the handpiece (1) that comprises a material which is part of the thermocouple (13), **characterized in that** the component whose material is part of the thermocouple (13) is formed by a contact ring (14) which is connected to the outer sleeve (2) in an electrically conductive manner, wherein the contact ring (14) is provided in particular on an actuation element (21) for actuating the tool release device (7).

4. Medical, in particular dental handpiece (1) according to any one of the preceding Claims, **characterized in that**
the handpiece (1) comprises a handpiece head (16) in which are provided a tool holder (15) for the tool which is connectible to the handpiece (1) and the at least one thermocouple (13).

5. Medical, in particular dental handpiece (1) according to Claim 2 or 3, **characterized in that**
the handpiece (1) comprises a handpiece head (16) and wherein the bearing (5) or the contact ring (14) are arranged in the handpiece head (16).

6. Medical, in particular dental handpiece (1) according to any one of the preceding Claims, **characterized in that**
the at least one thermocouple (13) is arranged in an interior (11) of the handpiece (1), wherein the interior (11) is formed by a volume (11') comprising a fluid, in particular air, and separates components of the handpiece (1).

7. Medical, in particular dental handpiece (1) according to any one of the preceding Claims, **characterized by**
an electrical line (22) which conducts the thermoelectric voltage signal or the measured signal of the at least one thermocouple (13) to a unit (23), which determines the temperature value on the basis of the received signal.

8. Medical, in particular dental handpiece (1) according to Claim 7, **characterized in that** the electrical line (22) comprises a first and second electrical conductor (22', 22") for conducting the thermoelectric voltage signal or the measured signal of the at least one thermocouple (13) to the unit (23), wherein at least one of the electrical conductors (22') is connected to the thermocouple (13) via the outer sleeve (2) such, that the thermoelectric voltage signal or the measured signal can be transmitted from the thermocouple (13) via the outer sleeve (2) and the electrical conductor (22') to the unit (23).

9. Medical, in particular dental handpiece (1) according to Claim 7, **characterized in that** the electrical line (22) comprises a first and second electrical conductor (22', 22") for conducting the thermoelectric voltage signal or the measured signal of the at least one thermocouple (13) to the unit (23), wherein one of the conductors (22', 22") contacts the component whose material is part of the thermocouple (13), for example the bearing (5).

10. Medical, in particular dental handpiece (1) according to any one of the preceding Claims 3 - 9, **characterized in that**
the contact ring (14) is separated from the handpiece (1), in particular from the outer sleeve (2), by electric insulation (24), wherein the electric insulation (24) is interrupted at a contact area or a contact point (25), so that direct electric contact is established between the contact ring (14) having a first material, and the outer shell (2) having a second different material, wherein the direct electric contact of the first and second material forms the thermocouple (13) for measuring the temperature.

11. Medical, in particular dental handpiece (1) according to any one of the preceding Claims, **characterized in that**
at least one additional thermocouple (13) for the measurement of the temperature of the handpiece (1) or a part of the handpiece (1) is provided in the handpiece (1).

12. Medical, in particular dental handpiece (1) according to any one of the preceding Claims, **characterized in that**
at least one additional electrical temperature measuring device (12) is provided in the handpiece (1), in particular an electrical temperature measuring device (12) having a material whose electrical resistance or dynamic resistance is variable as a function of temperature.

13. Medical, in particular dental handpiece (1) according to any one of the preceding Claims 7 - 12, **characterized in that**
the unit (23) is configured as evaluation unit for evaluating the measured signal of the temperature measuring device (4) and / or as regulating or control unit for temperature-dependent regulation or control of the operation of the handpiece (1), for example, for interrupting the operation of the handpiece (1) on reaching or exceeding a predetermined temperature limit value, and/or as display unit for displaying the measured or determined temperature value or a temperature limit value.

## Revendications

1. Pièce à main médicale, en particulier dentaire (1), comprenant: une gaine extérieure (2), un dispositif d'entraînement (3) pour mettre en mouvement un outil pouvant être relié à la pièce à main (1) et un dispositif de mesure de température (4) pour mesurer la température de la pièce à main (1) ou d'une partie de la pièce à main (1), lequel comprend un dispositif de mesure de température électrique (12) qui présente au moins un thermocouple (13) réalisé, pour la mesure de la température, pour mettre à disposition un thermosignal électrique de tension, dans laquelle l'au moins un thermocouple (13) est prévu sur un élément de la pièce à main (1), lequel présente un matériau qui fait partie du thermocouple (13), **caractérisée en ce que** l'élément, dont le matériau fait partie du thermocouple (13), est formé par la gaine extérieure (2) ou par un élément, relié de façon électroconductrice à la gaine extérieure (2), de la pièce à main (1).

2. Pièce à main médicale, en particulier dentaire (1), comprenant: une gaine extérieure (2), un dispositif d'entraînement (3) pour mettre en mouvement un outil pouvant être relié à la pièce à main (1) et un dispositif de mesure de température (4) pour mesurer la température de la pièce à main (1) ou d'une partie de la pièce à main (1), lequel comprend un dispositif de mesure de température électrique (12) qui présente au moins un thermocouple (13) réalisé, pour la mesure de la température, pour mettre à disposition un thermosignal électrique de tension, dans laquelle l'au moins un thermocouple (13) est prévu sur un élément de la pièce à main (1), lequel présente un matériau qui fait partie du thermocouple (13), **caractérisée en ce que** l'élément, dont le matériau fait partie du thermocouple (13), est formé par un palier (5) de la pièce à main (1).

3. Pièce à main médicale, en particulier dentaire (1), comprenant: une gaine extérieure (2), un dispositif d'entraînement (3) pour mettre en mouvement un outil pouvant être relié à la pièce à main (1) et un dispositif de mesure de température (4) pour mesurer la température de la pièce à main (1) ou d'une partie de la pièce à main (1), lequel comprend un dispositif de mesure de température électrique (12) qui présente au moins un thermocouple (13) réalisé, pour la mesure de la température, pour mettre à disposition un thermosignal électrique de tension, dans laquelle l'au moins un thermocouple (13) est prévu sur un élément de la pièce à main (1), lequel présente un matériau qui fait partie du thermocouple (13), **caractérisée en ce que** l'élément, dont le matériau fait partie du thermocouple (13), est formé par une bague de contact (14) reliée de façon électroconductrice à la gaine extérieure (2), dans laquelle la bague de contact (14) est en particulier prévue au niveau d'un élément d'actionnement (21) pour actionner le dispositif de libération d'outil (7).

4. Pièce à main médicale, en particulier dentaire (1), selon l'une des revendications précédentes, **caractérisée en ce que**
la pièce à main (1) présente une tête de pièce à main (16) dans laquelle sont prévus un porte-outil (15) pour l'outil pouvant être relié à la pièce à main (1) et qu'au moins un thermocouple (13).

5. Pièce à main médicale, en particulier dentaire (1), selon la revendication 2 ou 3, **caractérisée en ce que**
la pièce à main (1) présente une tête de pièce à main (16) et que le palier (5) ou la bague de contact (14) est disposé(e) dans la tête de pièce à main (16).

6. Pièce à main médicale, en particulier dentaire (1), selon l'une des revendications précédentes, **caractérisée en ce que**
l'au moins un thermocouple (13) est disposé dans un espace intérieur (11) de la pièce à main (1), dans laquelle l'espace intérieur (11) est formé par un volume (11') contenant un fluide, en particulier de l'air, lequel sépare entre eux des éléments de la pièce à main.

7. Pièce à main médicale, en particulier dentaire (1), selon l'une des revendications précédentes, **caractérisée par**
une ligne électrique (22), laquelle conduit le thermosignal de tension ou le signal de mesure de l'au moins un thermocouple (13) vers une unité (23) laquelle, sur la base du signal reçu, détermine la valeur de température.

8. Pièce à main médicale, en particulier dentaire (1), selon la revendication 7, **caractérisée en ce que**
la ligne électrique (22) présente un premier et un deuxième conducteur électrique (22', 22") pour conduire le thermosignal de tension ou le signal de mesure de l'au moins un thermocouple (13) vers l'unité (23), dans laquelle au moins l'un des conducteurs électriques (22') est relié, via la gaine extérieure (2), au thermocouple (13) de manière à ce que le thermosignal de tension ou le signal de mesure puisse être transmis du thermocouple (13) via la gaine extérieure (2) et le conducteur électrique (22') à l'unité (23).

9. Pièce à main médicale, en particulier dentaire (1), selon la revendication 7, **caractérisée en ce que**
la ligne électrique (22) présente un premier et un deuxième conducteur électrique (22', 22") pour conduire le thermosignal de tension ou le signal de mesure de l'au moins un thermocouple (13) vers l'unité (23), dans laquelle l'un des conducteurs (22', 22") entre en contact avec l'élément, dont le matériau fait partie du thermocouple (13), par exemple le palier (5).

10. Pièce à main médicale, en particulier dentaire (1), selon l'une des revendications 3 - 9 précédentes, **caractérisée en ce que**
la bague de contact (14) est séparée, par une isolation électrique (24), de la pièce à main (1), en particulier de la gaine extérieure (2), dans laquelle l'isolation électrique (24) est interrompue au niveau d'un emplacement de contact ou un point de contact (25), de sorte qu'un contact électrique direct est établi entre la bague de contact (14) qui présente un premier matériau et la gaine extérieure (2) qui présente un deuxième matériau différent, dans laquelle le contact électrique direct du premier et deuxième matériau forme le thermocouple (13) pour la mesure de la température.

11. Pièce à main médicale, en particulier dentaire (1), selon l'une des revendications précédentes, **caractérisée en ce que**
dans la pièce à main (1), au moins un autre thermocouple (13) pour la mesure de la température de la pièce à main (1) ou d'une partie de la pièce à main (1) est prévu.

12. Pièce à main médicale, en particulier dentaire (1), selon l'une des revendications précédentes, **caractérisée en ce que**
dans la pièce à main (1), au moins un autre dispositif électrique de mesure de température (12) est prévu, en particulier un dispositif électrique de mesure de température (12) avec un matériau dont la résistance électrique ou la résistance dynamique est variable en fonction de la température.

13. Pièce à main médicale, en particulier dentaire (1), selon l'une des revendications 7-12, **caractérisée en ce que**
l'unité (23) est réalisée en tant qu'unité d'évaluation pour évaluer les signaux de mesure du dispositif de mesure de température (4) et/ou en tant qu'unité de réglage ou de commande pour le réglage ou la commande, en fonction de la température, du fonctionnement de la pièce à main (1), par exemple pour l'interruption du fonctionnement de la pièce à main (1) à l'atteinte ou au dépassement d'une valeur de seuil de température prédéfinie, et/ou en tant qu'unité d'affichage pour afficher la valeur de température mesurée ou déterminée ou d'une valeur de seuil de température.
